# EUROPEAN PATENT SPECIFICATION

(11) **EP 3 681 856 B1**
(45) Date of publication and mention of the grant of the patent: **27.10.2021**
(21) Application number: 18785749.5
(22) Date of filing: 13.09.2018
(51) Int. Cl.: C07C 211/63, A01N 39/00, A61P 31/00, A61P 31/04

(54) **COMPOUNDS HAVING PRESERVATIVE, ANTIMICROBIAL AND ANTISEPTIC ACTIVITY**
VERBINDUNGEN MIT KONSERVIERENDER, ANTIMIKROBIELLER UND ANTISEPTISCHER WIRKUNG
COMPOSÉS PRÉSENTANT UNE ACTIVITÉ DE CONSERVATION, UNE ACTIVITÉ ANTIMICROBIENNE ET UNE ACTIVITÉ ANTISEPTIQUE

(30) Priority: 15.09.2017 IT 201700103637
(43) Date of publication of application: 22.07.2020
(73) Proprietor: Universita' degli Studi di Milano, 20122 Milano (IT)
(72) Inventor: FUMAGALLI, Laura, 20133 Milano (IT); PICOZZI, Claudia, 20133 Milano (IT); REGAZZONI, Luca, 20133 Milano (IT); CARINI, Marina, 20133 Milano (IT); ALDINI, Giancarlo, 20133 Milano (IT); VISTOLI, Giulio, 20133 Milano (IT)
(74) Representative: Bianchetti & Minoja SRL
(86) International application number: PCT/IB2018/057007
(87) International publication number: WO 2019/053626

(56) References cited:
- GB-A- 1 582 774
- KANJI YAMAMOTO ET AL: "Studies on the Inverted Soaps as the Fungicide", NIPPON NOGEI KAGAKUKAISHI - JOURNAL OF THE AGRICULTURAL CHEMICAL SOCIETY OF JAPAN, vol. 26, no. 11, 1 January 1952 (1952-01-01), pages 589-594, XP055464273, JP ISSN: 0002-1407, DOI: 10.1271/nogeikagaku1924.26.589
- MOTTA G ET AL: "Further studies on durene derivatives. III. New ammonium salts with disinfectant activities", BOLLETTINO CHIMICO FARMACEUTICO, SOC. ED. FARMACEUTICA, IT, vol. 118, no. 7, 1 January 1979 (1979-01-01), pages 373-9, XP009504518, ISSN: 0006-6648

## Description

### Technical field of invention

The present invention relates to domiphen bromide derivatives of formula (**I**)): 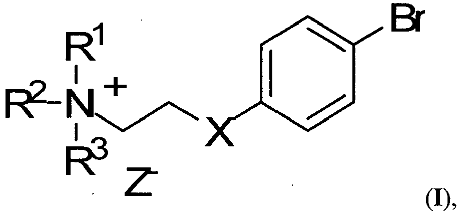 which have preservative, antimicrobial and antiseptic activities.

### Prior art

Domiphen bromide is a well-known quaternary ammonium salt used as an antimicrobial and preservative in various cosmetic and pharmaceutical preparations.

In the last few decades domiphen bromide has been described as both an active ingredient and a preservative excipient, for example in US2007/237726 A1 and WO2007/134003 A2, wherein domiphen bromide is described as an ingredient of oral hygiene formulations.

Domiphen bromide analogues having microbicidal and fungicidal activities, wherein the phenyl ring is substituted with at least one bromine atom and a further substituent, are disclosed, for example, in US3801641 A.

DE 1811515 discloses alkyldimethyl (phenoxyalkyl)ammonium halides having antispasmodic activity wherein a C12 alkyl is present and the phenyl ring is substituted at the para position by a chlorine or bromine atom and at the ortho position by a formyl group.

FR 2616065 A1 discloses aerosols with fungicidal, bactericidal and virucidal activity containing quaternary ammonium salts. The compounds cited include [2-(4-chlorophenoxy)ethyl](dodecyl)dimethylammonium bromide (dodeclonium bromide).

KANJI YAMAMOTO et al, (JOURNAL OF THE AGRICULTURAL CHEMICAL SOCIETY OF JAPAN, vol. 26, no. 11, pages 589-594) discloses dodecyl- and hexadecyl-based thioethers analogues of Domiphen with activity against *Giberella Fjikuroi,* a pathogen fungus for plants.

There is still a need to identify alternative compounds useful as preservatives and antimicrobials, especially for cosmetic and pharmaceutical products, and as antiseptics.

### List of figures

Figure 1 shows the ¹H-NMR spectrum of the compound of formula (**Ia**) (para-bromo domiphen bromide) in DMSO-d₆ at 28°C.
Figure 2 shows the ¹³C-NMR spectrum of the compound of formula (**Ia**) (para-bromo domiphen bromide) in DMSO-d₆ at 28°C.
Figure 3 shows the mass spectrum of the compound of formula (**Ia**) (para-bromo domiphen bromide).
Figure 4 shows the mass/mass fragmentation spectrum of the molecular ion relating to compound (**Ia**) (para-bromo domiphen bromide) having m/z 412.12.
Figure 5 shows the chromatogram relating to the HPLC analysis of compound (**Ia**) (para-bromo domiphen bromide).
Figure 6 shows the antimicrobial effect of domiphen bromide towards *E*. *coli* in the disc diffusion test.
Figure 7 shows the antimicrobial effect of the compound of formula (**Ia**) (para-bromo domiphen bromide) towards *Escherichia coli* in the disc diffusion test.
Figure 8 shows the antimicrobial effect of domiphen bromide towards *Staphylococcus aureus* in the disc diffusion test.
Figure 9 shows the antimicrobial effect of the compound of formula (**Ia**) (para-bromo domiphen bromide) towards *Staphylococcus aureus* in the disc diffusion test.
Figure 10 shows the antimicrobial effect of domiphen bromide towards *Candida humilis* in the disc diffusion test.
Figure 11 shows the antimicrobial effect of the compound of formula (**Ia**) (para-bromo domiphen bromide) towards *Candida humilis* in the disc diffusion test.

### Summary of the invention

The present invention relates to a compound having general formula (**I**):

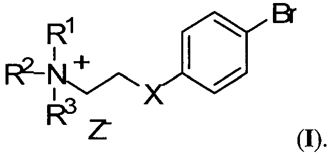

The invention also relates to the use of a compound of formula (**I**) as a preservative and antimicrobial.

A further object of the present invention is the use of a compound of formula (**I**) as a medicament, in particular as an antiseptic.

### Description of the invention

The object of the invention is a compound having general formula (**I**): 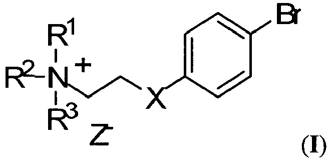 wherein
R¹ is an alkyl group with 8 to 18 carbon atoms;
R² is an alkyl group with 1 to 5 carbon atoms;
R³ is an alkyl group with 1 to 5 carbon atoms;
X is an oxygen or sulphur atom, preferably an oxygen atom;
Z⁻ is an inorganic or organic anion;
provided that when R¹ is a dodecyl or hexadecyl group, R² is methyl, R³ is methyl, Z⁻ is a chloride anion, and X is not sulphur.

According to a preferred aspect, the compound of formula (**I**) is (dodecyldimethyl-2-p-bromophenoxyethyl)ammonium bromide of formula: 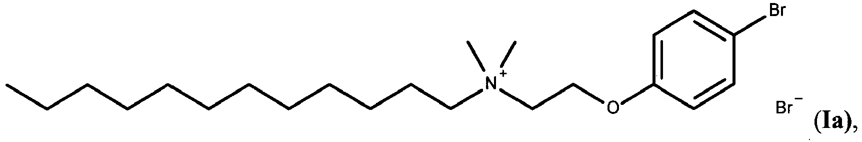 hereinafter also referred to as para-bromo domiphen bromide or bromiphen bromide.

The compounds of general formula (**I**) can be obtained by synthesis methods known in the prior art.

The compound of formula (**Ia**) is the mono-bromo analogue substituted at the para position of domiphen bromide, and can be obtained by reacting 2-(p-bromophenoxyethyl)-N,N-dimethylamine of formula (II) with bromododecane,

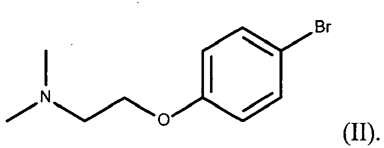

The reaction can be effected in a solvent preferably selected from dichloromethane and butanone, or in the absence of solvent, at a temperature preferably ranging from 30°C to 50°C, for a time preferably ranging from 4 hours to 6 hours.

In a preferred embodiment, the reaction is effected in the absence of solvent, at a temperature of 100°C for two hours.

The compound of formula (II) can be obtained by alkylation of p-bromophenol with 2-chloro-N,N-dimethylethylamine hydrochloride in the presence of a solvent preferably selected from dimethylformamide, ethanol, butanone and acetone, at a temperature preferably ranging from 40°C to 60°C, operating in the presence of an alkali metal iodide and carbonate, for a time preferably ranging from 8 hours to 10 hours.

In a preferred embodiment, the reaction is effected in 2-butanone at reflux for four hours in the presence of caesium carbonate and sodium iodide.

It has surprisingly been found that the compounds of formula (I) of the invention have antimicrobial activity towards microorganisms, such as Gram-positive and Gram-negative bacteria, and yeasts.

In particular, the compounds of formula (**I**) have antimicrobial activity comparable to that of domiphen bromide against *Escherichia coli* and *Staphylococcus aureus,* microorganisms which are Gram-negative and Gram-positive respectively, and also against the yeast *Candida humilis.* Moreover, further tests have proved that the compound of formula (**Ia**) is more active than domiphen against *Pseudomonas aeruginosa* (Gram-negative) and *Bacillus cereus* (Gram-positive).

A further object of the present invention is therefore the use of the compounds of formula (**I**), especially a compound of formula (**Ia**), as preservatives and antimicrobials, for example against Gram-negative and Gram-positive microorganisms and yeasts, such as *Staphylococcus aureus, Escherichia coli, Pseudomonas aeruginosa, Bacillus cereus* and *Candida humilis,* to preserve foodstuffs (especially food packaging), cosmetics and pharmaceuticals against microbial contamination, and/or to inhibit the development of microorganisms in said products.

A further object of the present invention is the use of the compounds of formula. (**I**), in particular a compound of formula (**Ia**), as a medicament, in particular as an antiseptic. The compounds of formula (**I**), in particular the compound of formula (**Ia**), are also useful in the treatment of infections caused by *Staphylococcus aureus, Escherichia coli* and *Pseudomonas aeruginosa.*

Unlike domiphen bromide, the compounds of formula (**I**), especially the compound of formula (**Ia**), are chemically stable. Unlike formulations containing domiphen bromide, whose domiphen bromide content tends to decline gradually with time, the formulations containing the compounds of formula (**I**), especially the compound of formula (**Ia**), are therefore stable, thus ensuring that the storage specifications of the preparations are always fulfilled.

Compositions containing the compounds of formula (**I**), especially the compound of formula (**Ia**), can be prepared by conventional methods known to the skilled person, and constitute a further object of the present invention.

The invention is further illustrated by the following examples.

### Example 1 - Synthesis of the compound of formula (Ia) (para-bromo domiphen bromide)

Caesium carbonate (3.0 Eq), sodium iodide (0.14 Eq) and 2-chloro-N,N-dimethylethylamine hydrochloride (1.5 Eq) are added to a suspension of *p*-bromo-phenol (1.0 Eq) in butanone (50 mL). The suspension is kept under reflux for four hours, after which the solid is filtered through a Hirsch funnel and washed with acetone, and the solvent is evaporated at low pressure. The crude product (yellow oil) is purified by silica-gel chromatography (eluent: dichloromethane-methanol; 95:5) to obtain the desired product (2-*p*-bromophenoxyethyl-N,N-dimethylamine) as a colourless oil (yield 70%).

2-*p*-Bromophenoxyethyl-N,N-dimethylamine (1.0 Eq) is then reacted with bromododecane (1.5 Eq) for two hours, heating at 100°C. At the end of the reaction the crude product is resuspended in acetone, from which bromiphen bromide precipitates as a white solid (yield 50%).

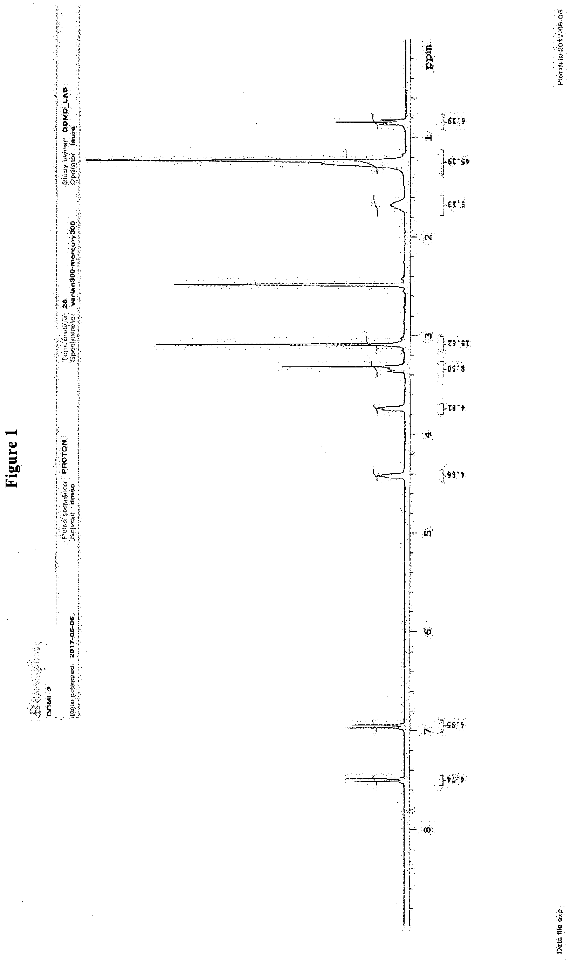

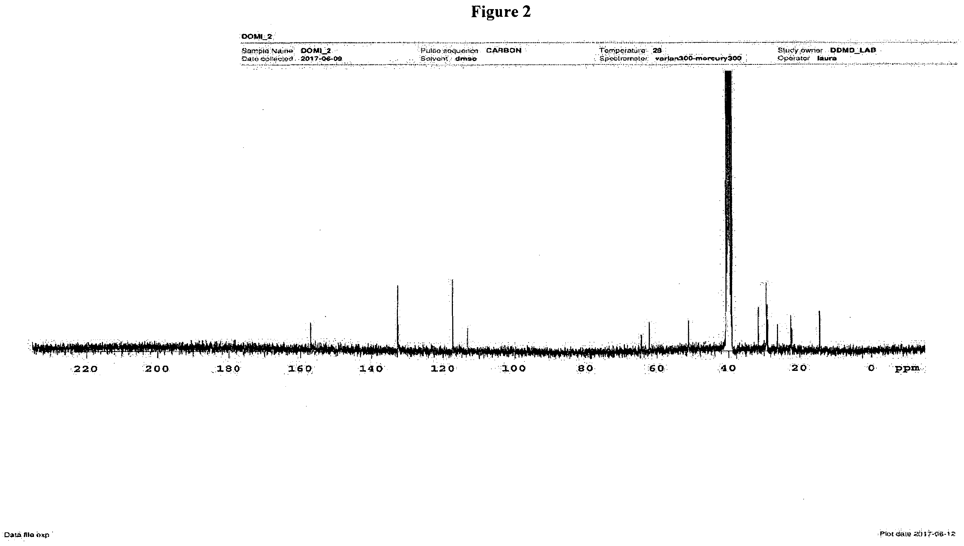

The ¹H-NMR and ¹³C-NMR spectra of the compound of formula (I) are shown in figures 1 and 2 respectively.

The mass spectrum of bromiphen bromide was recorded under the following conditions:
- MS detector
- Induced dissociation: 30%.
- Source: Electron spray ionisation (ESI), room temperature, N₂ 60 units.
- Collision gas: argon.
- Analyser: triple quadrupole (TSQ quantum, Thermo Scientific).

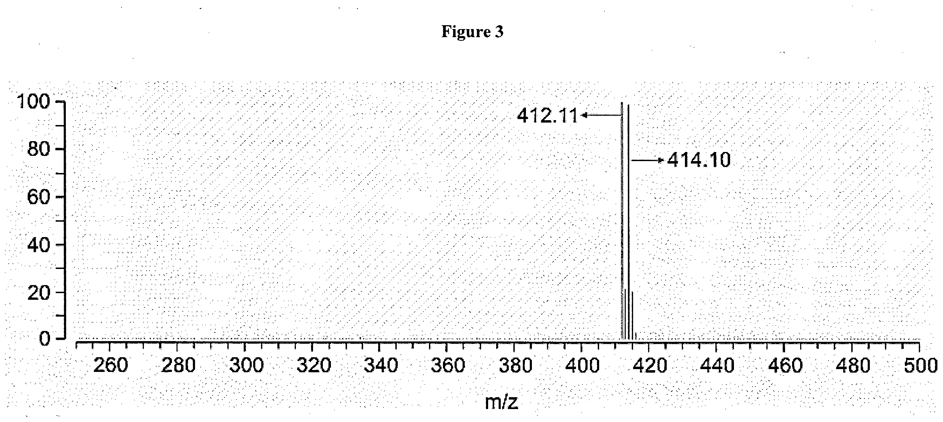

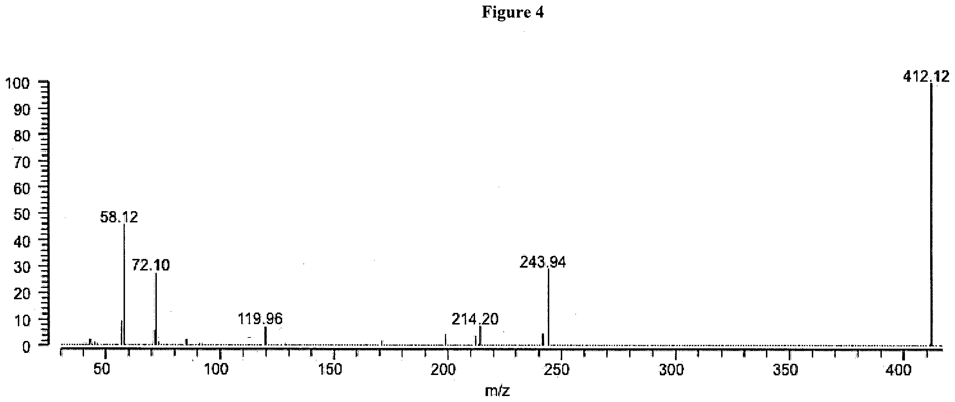

The mass spectrum of bromiphen bromide shows the molecular ions m/z+ 412.12 and 414.10 with the relative abundance typical of bromine (Figure 3). The mass-mass spectrum is shown in Figure 4.

The HPLC analysis of bromiphen bromide was effected under the following conditions:
COLUMN: 5 µm XBridgeTM C18 4.6 X 150 mm
GRADIENT
Eluent A: Acetonitrile R
Eluent B: buffer solution pH=4 (ammonium formate/formic acid)

| **Time** | **% Eluent A** | **% Eluent B** | **Flow rate** |
|---|---|---|---|
| 0 | 65 | 35 | 1.0 mL/min |
| 10 | 65 | 35 | 1.0 mL/min |
| 12 | 90 | 10 | 2.0 mL/min |
| 18 | 90 | 10 | 2.0 mL/min |
| 20 | 65 | 35 | 1.0 mL/min |
| 25 | 65 | 35 | 1.0 mL/min |

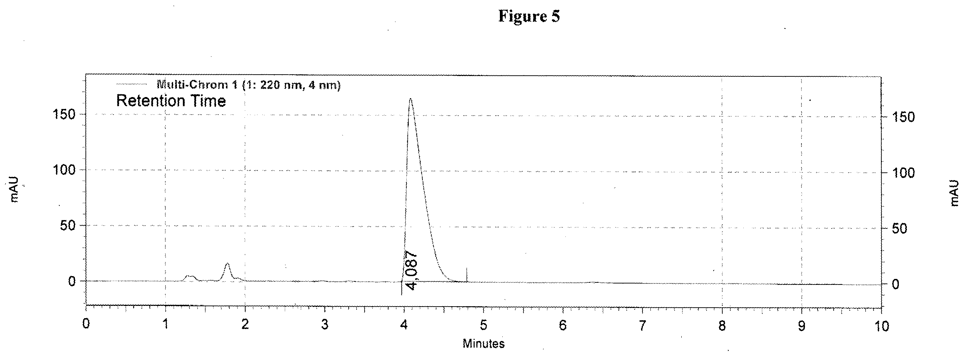

Flow rate: 1.0 mL/min;
Detector: diode array;
Column temperature: 40°C;
Injection volume: 20 µL;
Analysis time: 15 minutes.
The HPLC chromatogram recorded at 220 nm is shown in Figure 5.

### Example 2 - Bioassays

The antimicrobial susceptibility tests were effected by the disc diffusion method as indicated by EUCAST (European Committee on Antimicrobial Susceptibility Testing). The plates were prepared with Mueller Hinton Agar (MHA) medium into which microbial suspensions of *Escherichia coli* ATCC25922 and *Staphylococcus aureus* ATCC25923 were inoculated at a concentration of about 2×10⁸ CFU/mL. Sterile discs were then placed on each plate and subsequently inoculated with successive concentrations of the two chemical compounds analysed (domiphen bromide and para-bromo domiphen bromide), together with a positive control (chloramphenicol) and a negative control (MH broth).

The plates were then incubated at 37°C in aerobiosis for 16-20 h.

After incubation, any inhibition zone due to the potential antimicrobial activity was measured with a ruler, and the activities of the two molecules were compared.

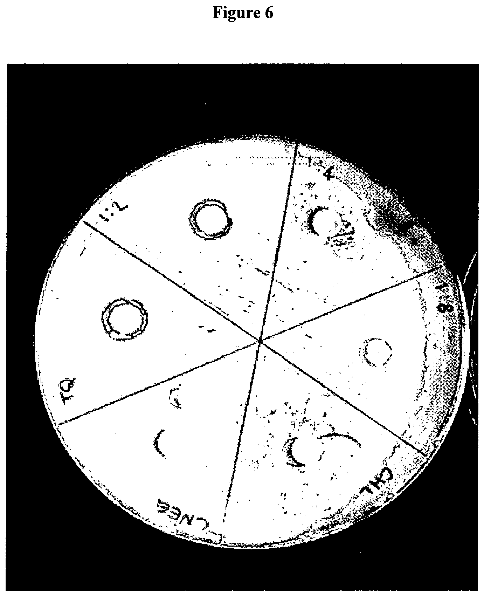

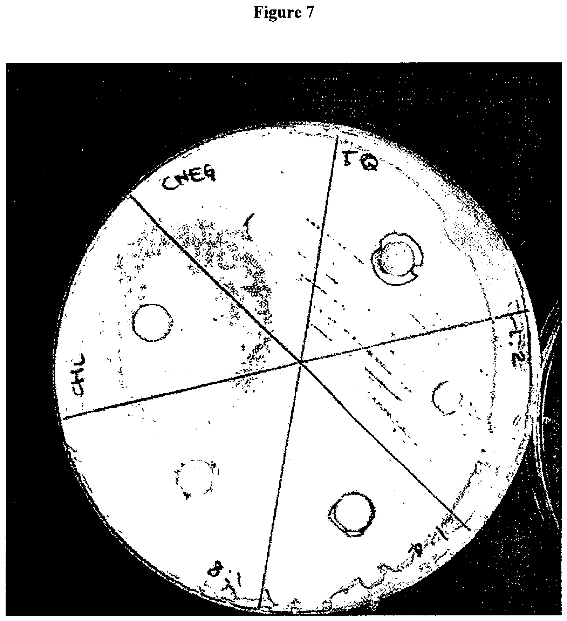

As shown by Figures 6 and 7, the antimicrobial activity of para-bromo domiphen bromide towards *E. coli* is comparable with that of domiphen bromide, exhibiting a slightly greater inhibition zone for para-bromo domiphen bromide.

In Figure 6 and Figure 7:
TQ= concentration of compound amounting to 1000 µg/mL 1:2= concentration of compound amounting to 500 µg/mL
1:4= concentration of compound amounting to 250 µg/mL
1:8= concentration of compound amounting to 125 µg/mL
CHL= chloramphenicol (antibiotic)
CNEG= negative control (MHB).

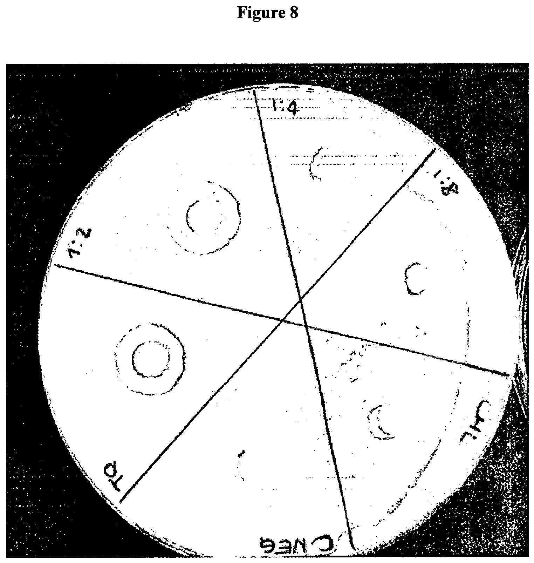

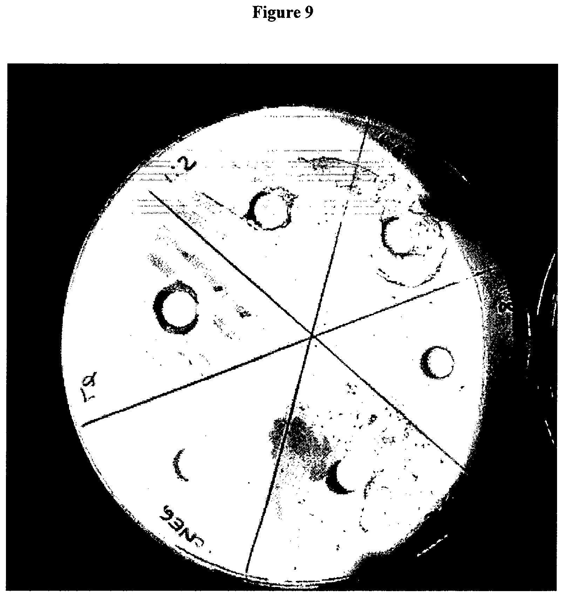

In the case of *S*. *aureus,* it will be seen (Figures 8 and 9) that para-bromo domiphen bromide also exhibits antimicrobial activity against said microorganism.

In Figure 8 and Figure 9:
TQ= concentration of compound amounting to 1000 µg/mL
1:2= concentration of compound amounting to 500 µg/mL
1:4= concentration of compound amounting to 250 µg/mL
1:8= concentration of compound amounting to 125 µg/mL
CHL= chloramphenicol (antibiotic)
CNEG= negative control (MHB).

### Example 3 - Bioassays - MIC evaluation

Tests were then effected on the two bacteria analysed with the agar diffusion test and on two other microorganisms such as *Pseudomonas aeruginosa* DSM 22644 (PAO1) and *Bacillus cereus* DSM31 to evaluate the Minimum Inhibitory Concentration (MIC), i.e. the lowest concentration of compound able to inhibit the growth of a microorganism. The method used was the broth microdilution method (CLSI - Clinical and Laboratory Standards Institute). Briefly, the procedure involved the preparation of serial dilutions of the antimicrobial agent (256 to 0.125 mg/1) distributed in a 96-well microtiter plate (microdilution). Each well was then inoculated with the reference microbial strain at the concentration of about 5×10⁶ CFU/mL (0.5 McFarlands). The 96-well microtiter plates were then incubated at 37°C.

The MIC obtained for *E. coli* and *S. aureus* confirmed the results of the disc diffusion tests, giving a value of 8 mg/L for *E. coli* and 1 mg/L for *S. aureus* for both compounds. In the case of *P. aeruginosa* and *B. cereus,* however, the compound of formula (**Ia**) proved to be more effective, with a MIC value of 0.5 mg/mL vs. 1 mg/mL for domiphen against *B. cereus,* and 4 mg/mL vs. 8 mg/mL against *P. aeruginosa.*

### Example 4 - Bioassays - yeasts

Some preliminary tests were also effected to evaluate the activity against eukaryotic microorganisms such as yeasts.

The disc diffusion method was applied again, using the yeast *Candida humilis* UMY12 (UNIMI collection).

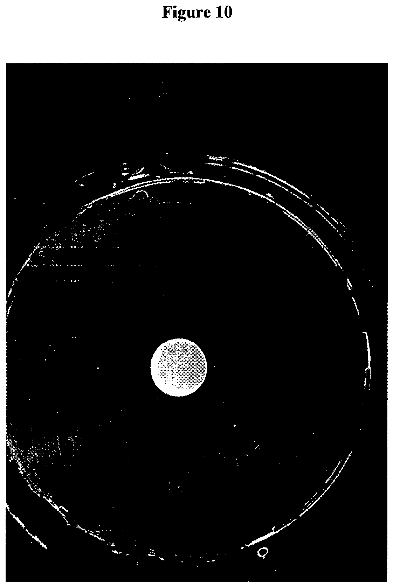

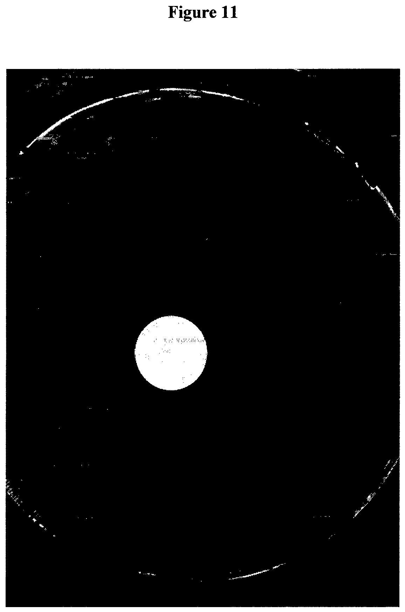

2% glucose was added to the MHA medium, and the concentration of the suspension amounted to about 3×10⁶ CFU/mL. The concentration of the chemical compounds amounted to 5120 µg/mL for both compounds. As will be seen from Figures 10 and 11, para-bromo domiphen and domiphen exhibit almost the same antimicrobial activity against yeast.

## Claims

1. A compound of general formula (I): 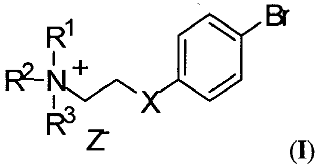 wherein
R¹ is an alkyl group with 8 to 18 carbon atoms;
R² is an alkyl group with 1 to 5 carbon atoms;
R³ is an alkyl group with 1 to 5 carbon atoms;
X is an oxygen or sulphur atom;
Z⁻ is an inorganic or organic anion;
provided that when R¹ is a dodecyl or hexadecyl group, R² is methyl, R³ is methyl,
Z⁻ is a chloride anion, and X is not sulphur.

2. The compound according to claim 1, which is (dodecyldimethyl-2-p-bromophenoxyethyl)ammonium bromide of formula (**Ia**):

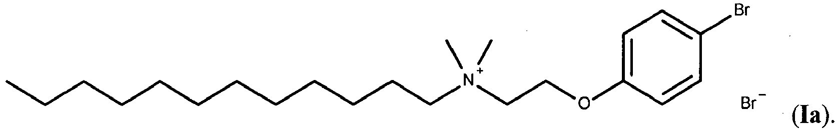

3. The use of a compound of formula (**I**) or (**Ia**) according to claim 1 or 2 as a preservative.

4. The use of a compound of formula (**I**) or (**Ia**) according to claim 1 or 2 as an antimicrobial for inhibiting the development of microorganisms in the packaging of food products, and in cosmetic and pharmaceutical products.

5. The use according to claim 4, wherein the antimicrobial compound is active against Gram-positive and Gram-negative bacteria, and yeasts.

6. The use according to claim 5, wherein the Gram-positive and Gram-negative bacteria are *Staphylococcus aureus, Escherichia coli, Bacillus cereus* and *Pseudomonas aeruginosa.*

7. The use according to claim 5, wherein the yeast is *Candida humilis.*

8. A compound of general formula (**I**) or (**Ia**) according to claim 1 or 2 for use as a medicament.

9. A compound for the use according to claim 8, as an antiseptic agent.

10. A compound for the use according to claim 8, in the treatment of infections induced by *Staphylococcus aureus, Escherichia coli* and *Pseudomonas aeruginosa.*

11. A composition comprising a compound of formula (**I**) according to claim 1 or a compound of formula (**Ia**) according to claim 2, as a preservative and antimicrobial or as an antiseptic agent, and at least one further suitable ingredient.

## Patentansprüche

1. Verbindung der allgemeinen Formel (I): 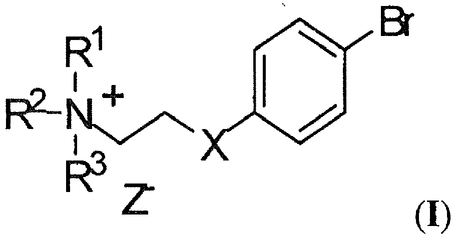 wobei:
R¹ eine Alkylgruppe mit 8 bis 18 Kohlenstoffatomen ist;
R² eine Alkylgruppe mit 1 bis 5 Kohlenstoffatomen ist;
R³ eine Alkylgruppe mit 1 bis 5 Kohlenstoffatomen ist;
X ein Sauerstoff- oder Schwefelatom ist;
Z⁻ ein anorganisches oder organisches Anion ist;
unter der Bedingung, dass, wenn R¹ eine Dodecyl- oder Hexadecylgruppe ist, R² Methyl ist, R³ Methyl ist,
Z⁻ ein Chloridanion ist und X kein Schwefel ist.

2. Verbindung gemäß Anspruch 1, welche (Dodecyldimethyl-2-p-brom-phenoxyethyl)ammoniumbromid der Formel (**Ia**) ist:

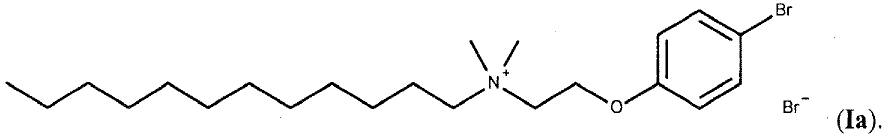

3. Verwendung einer Verbindung der Formel (**I**) oder (**Ia**) gemäß Anspruch 1 oder 2 als Konservierungsmittel.

4. Verwendung einer Verbindung der Formel (**I**) oder (**Ia**) gemäß Anspruch 1 oder 2 als antimikrobielles Mittel zur Inhibierung der Entwicklung von Mikroorganismen bei der Verpackung von Lebensmittelprodukten und bei kosmetischen und pharmazeutischen Produkten.

5. Verwendung gemäß Anspruch 4, wobei die antimikrobielle Verbindung aktiv gegen grampositive und gramnegative Bakterien und Hefen ist.

6. Verwendung gemäß Anspruch 5, wobei die grampositiven und gramnegativen Bakterien *Staphylococcus aureus, Escherichia coli, Bacillus cereus* und *Pseudomonas aeruginosa* sind.

7. Verwendung gemäß Anspruch 5, wobei die Hefe *Candida humilis* ist.

8. Verbindung der allgemeinen Formel (**I**) oder (**Ia**) gemäß Anspruch 1 oder 2 zur Verwendung als Medikament.

9. Verbindung zur Verwendung gemäß Anspruch 8 als antiseptisches Agens.

10. Verbindung zur Verwendung gemäß Anspruch 8 bei der Behandlung von Infektionen, welche durch *Staphylococcus aureus, Escherichia coli* und *Pseudomonas aeruginosa* verursacht sind.

11. Zusammensetzung, umfassend eine Verbindung der Formel (**I**) gemäß Anspruch 1 oder eine Verbindung der Formel (**Ia**) gemäß Anspruch 2 als Konservierungsmittel und antimikrobielles Mittel oder als antiseptisches Agens, und mindestens einen zusätzlichen geeigneten Inhaltsstoff.

## Revendications

1. Composé de formule générale (I) : 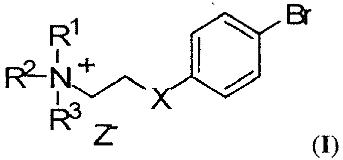 dans lequel
R¹ est un groupe alkyle avec 8 à 18 atomes de carbone ;
R² est un groupe alkyle avec 1 à 5 atomes de carbone ;
R³ est un groupe alkyle avec 1 à 5 atomes de carbone ;
X est un atome d'oxygène ou de soufre ;
Z⁻ est un anion inorganique ou organique ;
sous réserve que lorsque R¹ est un groupe dodécyle ou hexadécyle, R² est un méthyle, R³ est un méthyle, Z⁻ est un anion chlorure, et X n'est pas le soufre.

2. Composé selon la revendication 1, qui est le bromure de (dodécyldiméthyl-2-p-bromophénoxyéthyl)ammonium de formule (la) :

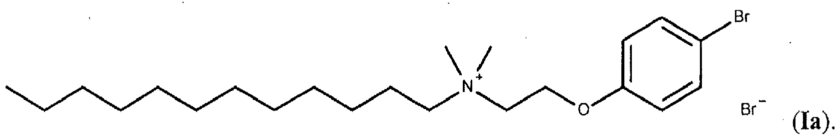

3. Utilisation d'un composé de formule (**I**) ou (**Ia**) selon la revendication 1 ou 2 en tant que conservateur.

4. Utilisation d'un composé de formule (**I**) ou (**Ia**) selon la revendication 1 ou 2 en tant qu'antimicrobien pour inhiber le développement de microorganismes dans le conditionnement des produits alimentaires, et dans les produits cosmétiques et pharmaceutiques.

5. Utilisation selon la revendication 4, dans laquelle le composé antimicrobien est actif contre les bactéries à Gram positif et à Gram négatif, et les levures.

6. Utilisation selon la revendication 5, dans laquelle les bactéries à Gram positif et à Gram négatif sont *Staphylococcus aureus, Escherichia coli, Bacillus cereus* et *Pseudomonas aeruginosa.*

7. Utilisation selon la revendication 5, dans laquelle la levure est *Candida humilis.*

8. Composé de formule générale (**I**) ou (**Ia**) selon la revendication 1 ou 2 destiné à être utilisé en tant que médicament.

9. Composé destiné à être utilisé selon la revendication 8, en tant qu'agent antiseptique.

10. Composé destiné à être utilisé selon la revendication 8, dans le traitement d'infections induites par *Staphylococcus aureus, Escherichia coli* et *Pseudomonas aeruginosa.*

11. Composition comprenant un composé de formule (**I**) selon la revendication 1 ou un composé de formule (**Ia**) selon la revendication 2, en tant que conservateur et antimicrobien ou en tant qu'agent antiseptique, et au moins un ingrédient approprié supplémentaire.
